# EUROPEAN PATENT SPECIFICATION

(11) **EP 1 855 596 B1**
(45) Date of publication and mention of the grant of the patent: **01.07.2015**
(21) Application number: 06710924.9
(22) Date of filing: 16.02.2006
(51) Int. Cl.: A61B 8/08, A61B 8/00, A61B 5/00

(54) **ULTRASONIC DIAGNOSTIC IMAGING SYSTEM FOR DETECTING LESIONS OF THE LIVER**
DIAGNOSTISCHES ULTRASCHALLSYSTEM ZUM NACHWEIS VON LEBERLÄSIONEN
SYSTEME D'IMAGERIE DE DIAGNOSTIC ULTRASONORE DE DETECTION DE LESIONS HEPATIQUES

(30) Priority: 23.02.2005 US 655824 P
(43) Date of publication of application: 21.11.2007
(73) Proprietor: Koninklijke Philips N.V., 5656 AE Eindhoven (NL)
(72) Inventor: BRUCE, Matthew, Bothell, WA 98041-3003 (US); POWERS, Jeffry, Bothell, WA 98041-3003 (US); GARG, Rohit, Bothell, WA 98041-3003 (US); SKYBA, Dan, Bothell, WA 98041-3003 (US); AVERKIOU, Michalakis, Bothell, WA 98041-3003 (US)
(74) Representative: Verweij, Petronella Danielle
(86) International application number: PCT/IB2006/050512
(87) International publication number: WO 2006/090309

(56) References cited:
- US-A- 2002 103 437
- US-A- 2004 120 559
- US-A1- 2002 035 326
- US-A1- 2002 035 329
- US-A1- 2002 045 823
- US-A1- 2003 073 903
- US-B1- 6 620 103
- AVERKIOU MICHALAKIS ET AL: "Ultrasound contrast imaging research." ULTRASOUND QUARTERLY. MAR 2003, vol. 19, no. 1, March 2003 (2003-03), pages 27-37, XP008067496 ISSN: 0894-8771 cited in the application

## Description

This invention relates to ultrasonic diagnostic imaging systems and, in particular, to ultrasound systems and methods for diagnosing lesions of the liver.

Hepatitis B and hepatitis C patients have been found to be at an increased risk of developing primary liver cancer, hepatocellular carcinoma (HCC). Due to the late discovery that hepatitis C was being contracted by patients through blood transfusions in the early 1980's, there remain a significant number of hepatitis C patients who need to be examined regularly for the onset of HCC, as the lesions are best treated in their early stages. The usual progression of the disease is from hepatitis to liver cirrhosis to HCC. An easy-to-use monitoring technique for liver disease progression would have widespread application in assisting in the early detection of this serious disease.

There are a number of lesions in addition to HCC which can develop in the liver. Our paper "Ultrasound Contrast Imaging Research" by M. Averkiou et al., published in Ultrasound Quarterly, vol. 19, no. 1 at pp 27 et seq. (2003) discusses four primary liver lesions: hepatocellular carcinoma (HCC), metastasis from a primary tumor at some other location, hemangioma, and focal nodal hyperplasia. The former two are malignant and the latter two are benign. A technique for identifying lesions in the liver should be able to detect all four of these lesions so that more detailed diagnosis can identify and distinguish between all lesions found in the liver.

US patent application serial number 60/542,259 entitled "ULTRASONIC IMAGING OF PERFUSION AND BLOOD FLOW WITH HARMONIC CONTRAST AGENTS," of which several of the present inventors are co-inventors, describes a technique for classifying or segmenting ultrasonic signals received during a contrast exam as to the anatomical sources of the signals. This information is used to distinctly depict tissue, capillary beds, and blood flow in larger vessels in the ultrasonic image. The ultrasonic signal information is segmented by using different filters for harmonic Doppler information produced by the signals, then classifying the differently filtered signals by velocity variance to determine whether the signals came from tissue, stationary contrast agent microbubbles in small capillaries, or flowing microbubbles in larger blood vessels. US patent 6,814,703 discusses a different technique for the same problem, classifying ultrasound signals by their Doppler power (P), B mode amplitude (B), and velocity (V) characteristics. Both patents show application of their techniques in liver diagnosis, and both rely on the fact that blood flow velocities in larger vessels such as arteries are greater than blood flow velocities in smaller vessels such as capillary beds. Both techniques thus require Doppler processing in order to determine signal segmentation. However, despite the complexities and versatilities of these techniques, neither patent addresses the unique problem of liver lesion detection and diagnosis.

US patent application publication 2002/0103437 relates to a blood flow imaging method by which a plurality of streams of blood flow having different contrast agent arrival times or blood concentration properties can be discerned.

In accordance with the principles of the present invention, pathology is detected during an ultrasonic contrast exam by segmentation on the basis of the time of arrival of the contrast agent at a particular anatomical site in the body. The location of anatomy exhibiting such a characteristic is uniquely identified in the ultrasound image as by a particular color or brightness. Time of arrival information may be correlated with signal amplitude in the classification process, and curve-fitting techniques may also be used. In one embodiment frame-to-frame motion of the anatomy is corrected for better detection. Embodiments of the present invention are particularly useful for detecting HCC and other lesions in the liver.

In the drawings:
FIGURE 1 illustrates in block diagram form an ultrasonic diagnostic imaging system constructed in accordance with the principles of the present invention.
FIGURES 2 and 3 illustrate comparison of pixel values over time for normal tissue and lesions in the liver.
FIGURE 4 is one embodiment of a signal classifier constructed in accordance with the principles of the present invention.
FIGURE 5 is another embodiment of a signal classifier constructed in accordance with the principles of the present invention.
FIGURE 6 is yet another embodiment of a signal classifier constructed in accordance with the principles of the present invention.
FIGURE 7 illustrates in block diagram form an ultrasonic diagnostic imaging system which aligns the image data for classification in accordance with the principles of the present invention.

Blood flow to the liver comes from two sources: the hepatic artery and the portal vein. The hepatic artery is supplied with blood flow from the aorta. The flow of blood through the portal vein comes from the inferior vena cava. The inferior vena cava in turn receives blood from the kidneys which cleanse a flow of blood supplied by the aorta. Blood flow out of the liver passes through the hepatic vein and to the superior vena cava. The liver receives 20% of its total supply of blood from the aorta and hepatic artery and 80% of its blood supply from the inferior vena cava and the portal vein.

As a result of this vasculature, when a bolus of contrast agent is injected into a vein, it will first begin to appear in the liver by way of the hepatic artery. After being pumped from the heart and into the aorta, some of the contrast agent will flow into the hepatic artery and begin to infuse the liver. But since only 20% of the blood supply to the liver is provided by the hepatic artery, this early infusion will be at a low level. The rest of the contrast agent flow in the aorta flows into the stomach and intestinal vasculature and then is collected in the portal vein which then significantly infuse the liver parenchyma.

It has been found that the vasculature developed by HCC lesions are supplied with their primary source of blood flow from the hepatic artery. Because the contrast agent first appears in the liver by way of the hepatic artery, this means that one of the initial sites of contrast agent detection in the liver after a bolus injection is the locus of an HCC lesion. If no lesion is present, only the usual hepatic artery and normal vessels supplied by it will appear with contrast present. After another 10-20 seconds the remainder of the bolus of contrast will have passed through the gastrointestinal system, the inferior vena cava, and the portal vein and this delayed flow of contrast agent will begin to appear as the capillary bed of the liver becomes infused with contrast agent. Since about 80% of the bolus will be delivered to the capillary bed, the delayed flow of contrast agent in the capillary bed will quickly overwhelm the appearance of the lesser supply of contrast in the HCC lesion and the HCC lesion will begin to appear dimmer in the ultrasound image in relation to the surrounding brightly appearing normal tissue of the capillary bed. There are thus only a few heartbeats and a few seconds in the initial infusion phase when the HCC lesion will distinctively stand out with contrast in the image, termed "early wash-in." It is this early wash-in characteristic which must be identified in the imaging procedure in the diagnosis of an HCC lesion, and there is only a small window of time in which to make it.

In accordance with the principles of the present invention the diagnosis of HCC lesions is enhanced by producing a parametric image of the liver based upon the relative arrival times of contrast agent in the liver to highlight areas of early wash-in which may be suspicious of HCC. The overall brightening of the liver with the arrival of contrast agent is monitored and areas that become bright early are identified. These areas are distinctively denoted in the image as by a unique brightness or color, indicating areas of suspicion for further investigation. The inventive technique is particularly suitable for 3D imaging where a significant volume of the liver can be diagnosed at one time. The 3D parametric image can be rendered from the parametric volume using a simple maximum intensity projection or other known or newly developed 3D imaging technique.

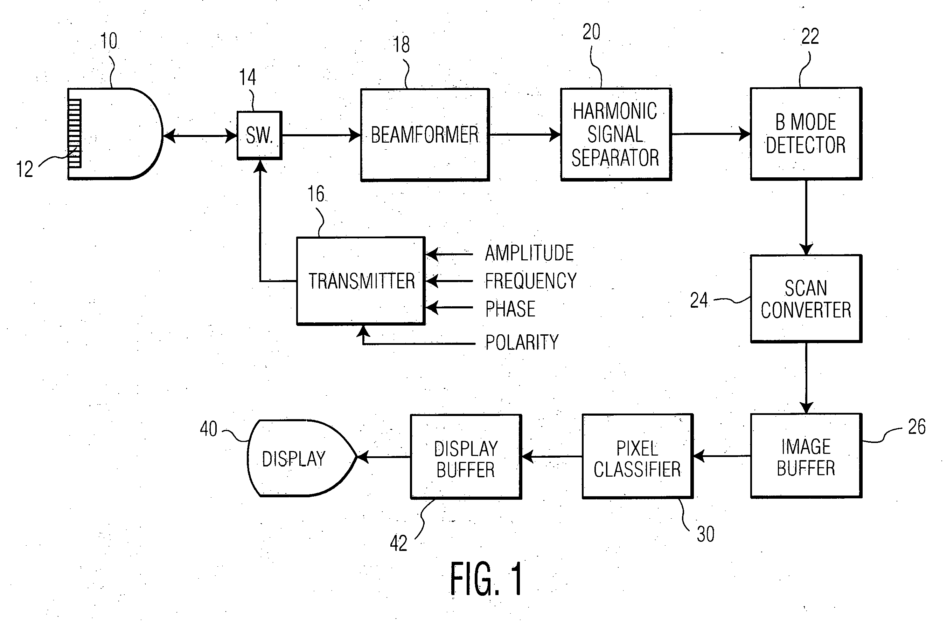

Turning first to FIGURE 1, an ultrasonic diagnostic imaging system constructed in accordance with the principles of the present invention is shown in block diagram form. This system operates by scanning a two or three dimensional region of the body being imaged with ultrasonic transmit beams. As each beam is transmitted along its steered path through the body, the tissue and blood flow in the body return echo signals with linear and nonlinear (or fundamental and harmonic) components corresponding to the transmitted frequency components. The transmit signals are reflected from the microbubbles of a contrast agent which exhibit a nonlinear response to ultrasound. The nonlinear response will cause the echo signals returned from the contrast agent to contain nonlinear components.

The ultrasound system of FIGURE 1 utilizes a transmitter 16 which transmits waves or pulses of a selected modulation characteristic in a desired beam direction for the return of harmonic echo components from scatterers within the body. The transmitter is responsive to a number of control parameters which determine the characteristics of the transmit beams as shown in the drawing, including the frequency components of the transmit beam, their relative intensities or amplitudes, and the phase or polarity of the transmit signals. The transmitter is coupled by a transmit/receive switch 14 to the elements of an array transducer 12 of a probe 10. The array transducer can be a one dimensional array for planar (two dimensional) imaging or a two dimensional array for two dimensional or volumetric (three dimensional) imaging.

The transducer array 12 receives echoes from the body containing linear and nonlinear components which are within the transducer passband. These echo signals are coupled by the switch 14 to a beamformer 18 which appropriately delays echo signals from the different transducer elements, then combines them to form a sequence of coherent echo signals along the beam from shallow to deeper depths. Preferably the beamformer is a digital beamformer operating on digitized echo signals to produce a sequence of discrete coherent digital echo signals from a near field to a far field depth of field. The beamformer may be a multiline beamformer which produces two or more sequences of echo signals along multiple spatially distinct receive scanlines in response to the transmission of one or more spatially distinct transmit beams, which is particularly useful for 3D imaging. The beamformed echo signals are coupled to a harmonic signal separator 20.

The harmonic signal separator 20 can separate the linear and nonlinear components of the echoes signal in various ways. One way is by filtering. Since certain nonlinear components such as the second harmonic are at a different frequency band (2fₒ) than the fundamental transmit frequencies (fₒ), the harmonic signals which are the signature of the contrast agent can be separated from the linear components by band pass or high pass filtering. There are also a number of multiple pulse techniques for separating nonlinear components which are generally referred to as pulse inversion techniques. In pulse inversion the image field is insonified by the transmission of multiple, differently modulated transmit signals in each beam direction, returning multiple echoes from the same location in the image field. The transmit signals may be modulated in amplitude, phase, polarity, or a combination thereof. When the received echoes from a common location are combined, the linear signal components are canceled and the nonlinear signal components reinforce each other (or *vice versa,* as desired), thereby producing separated nonlinear (e.g., harmonic) echo signals for imaging.

The echo signals are detected by a B mode detector 22. An advantage of the inventive technique over the prior art techniques discussed above is that Doppler processing is not necessary. The present invention may be carried out using Doppler processing if desired in a given embodiment, however the use of B mode signals avoids the reduction in real time frame rate caused by the acquisition of long Doppler ensembles. The detected echo signals are then converted into the desired image format such as a sector or pyramidal image by a scan converter 24. The scan converted image is temporarily stored in an image buffer 26 from which it can undergo further processing.

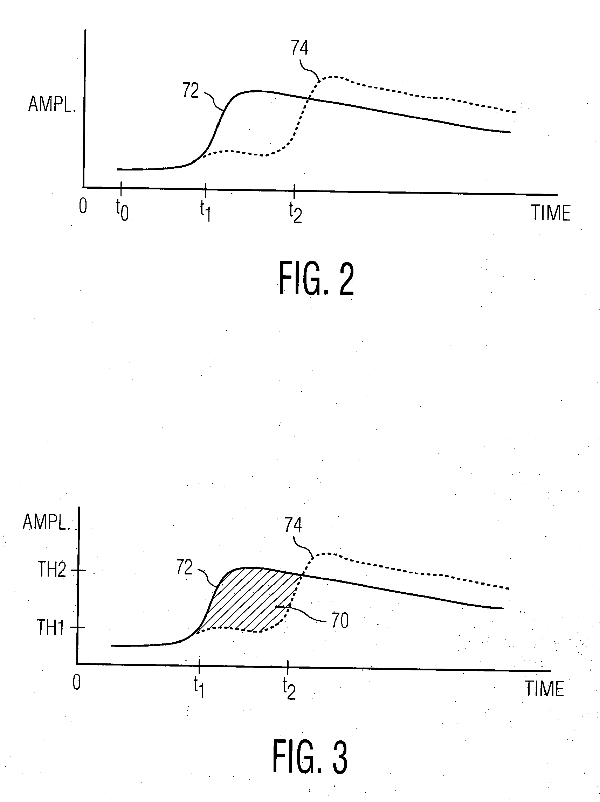

In accordance with the principles of the present invention a pixel classifier 30 classifies the pixels or voxels in the ultrasound image, looking for those that are characteristic of HCC lesions. FIGURE 2 gives a graphical appreciation of this analysis. Most of the flow of blood with contrast agent to normal tissue in the liver is delayed from the time of a bolus injection at time t₀ by its passage through the kidneys and thereafter its arrival at the liver at a time t₂ by way of the inferior vena cava (IVC), as illustrated by the dotted curve 74. There may be a small, perceptible increase in contrast in the normal tissue at time t₁ from the much smaller amount of blood with contrast agent delivered by the hepatic artery. However the flow of blood with contrast agent in the hepatic artery and into the vasculature developed around an HCC lesion will appear earlier than the main IVC flow as shown by the rise of solid line curve 72 at time t₁. The lesser flow delivered by the hepatic artery may result in achievement of a lesser concentration of contrast agent as indicated by the lower peak of the curve 72. It is therefore the function of the pixel classifier 30 to identify pixel or voxel data which is characteristic of the cross-hatched area 70 between curves 72 and 74, as shown in FIGURE 3, the early wash-in of contrast to the vasculature of an HCC lesion.

When the pixel classifier 30 identifies such data it highlights the subject pixels or voxels in a distinctive shade, brightness, or color. This creates a parametric image in which the parameter characteristic of suspect pathology is highlighted to the user. The parametric image is put into a display buffer 42, from which it is shown on a display 40.

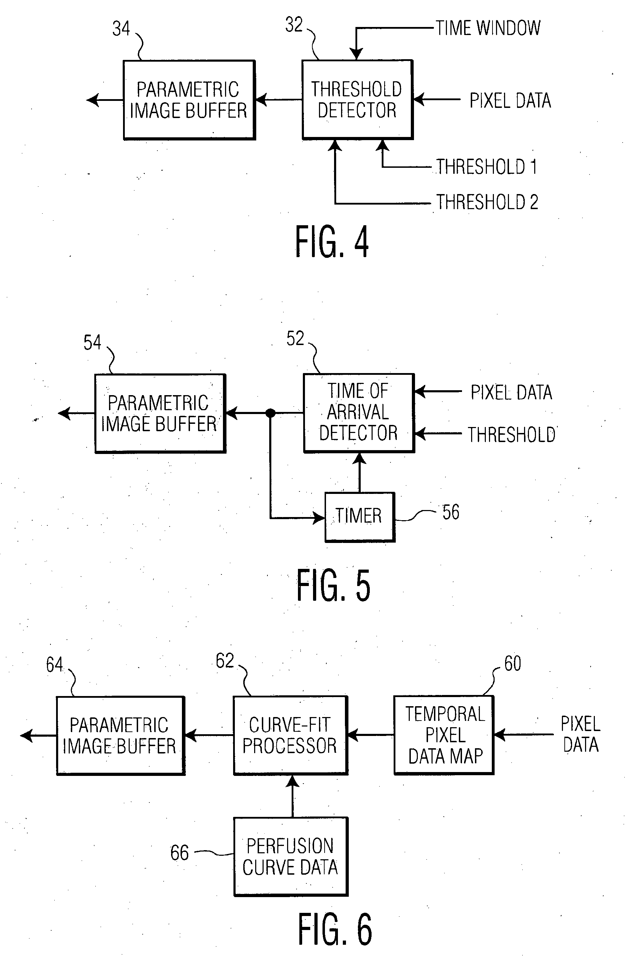

The pixel classifier 30 may operate in a variety of ways, one comparative example of which is illustrated in FIGURE 4. The pixel or voxel data from a succession of acquired images is applied to a threshold detector 32, which compares the levels of the image signals on a location-by-location basis. When a received pixel or voxel which is characteristic of the applied contrast agent, such as a second harmonic signal, exceeds a lower Threshold 1 during a given time window, such as the t₀-t₂ time interval in FIGURES 2-3, the pixel at that image location is classified as an early wash-in contrast pixel or voxel and is coded as such in the parametric image in the parametric image buffer 34. As signals at other pixel or voxel locations exceed the Threshold 1 level during the time window the liver image in the parametric image buffer builds up with a characteristic color or brightness in the hepatic artery and in the vicinity of any HCC lesions present. The build-up of the distinctive early wash-in of contrast can be observed in real time by periodically displaying the parametric image of the buffer, or the ultrasound system can wait until the time window closes and the build-up of early wash-in is completed before displaying the final early wash-in parametric image. The parametric image is limited to displaying only the desired early wash-in of contrast by closure of the time window before the later arrival of contrast from the IVC (i.e., before the rise of curve 74 in FIGURES 2-3), and by preventing the detection of signal levels above a higher Threshold 2 (see FIGURE#) which is a level anticipated to be achieved by only later-arriving contrast agent.

FIGURE 5 illustrates an embodiment of a pixel classifier according to the invention. In this embodiment pixel or voxel data from a succession of acquired images is compared with a Threshold, and those exceeding the Threshold (which are assumed to be above a noise level) are applied to a time of arrival detector 52. The time of arrival detector 52 is operative during the interval when a timer 56 is running. The timer 56 may be initiated by the first pixel value to exceed the Threshold, which would be anticipated to occur at time t₁ of FIGURE 3, and will time out after running for the t₁-t₂ interval. Thus, early signal levels characteristic of early wash-in pixels or voxels are identified during this time interval. If the identification of early wash-in pixels or voxels ended before the timing out of the timer 56, the timer would shut down earlier to prevent the detection of later-arriving contrast pixels or voxels by the time of arrival detector 52. As before, the early wash-in pixel or voxel locations identified are coded in the parametric image of the image buffer 54 and the parametric image is displayed either in real time as it is built up or after the build-up of early wash-in pixel or voxel areas is complete.

Another comparative example of a pixel classifier is shown in FIGURE 6. In this embodiment the pixel or voxel values of a succession of acquired images are stored in a temporal pixel data map 60 while the contrast agent begins to flow into and then recedes from the liver. The data map stores the temporal signal values for each spatially different location in the image field. The result is a succession of values at each location in the data map which, when plotted, may resemble a curve such as curves 72 and 74 in FIGURES 2-3. Each curve is analyzed by a curve-fit processor 62 which analytically compares the data curve at each pixel or voxel location with characteristic curve data stored in a perfusion curve data source 66. If the curve at a given pixel or voxel location fits characteristic curve 72, for instance, that location is classified as an early wash-in location in the image of the parametric image buffer 64. If the curve at a given location matches curve 74, that pixel or voxel location is classified as normal tissue. The result will be a parametric image developed in the buffer 64 which highlights early wash-in areas in the liver. This embodiment, like the others, may be constructed to analyze the whole image region to identify points where the liver experiences an initial lighting with contrast as compared with normal tissue in the rest of the liver image.

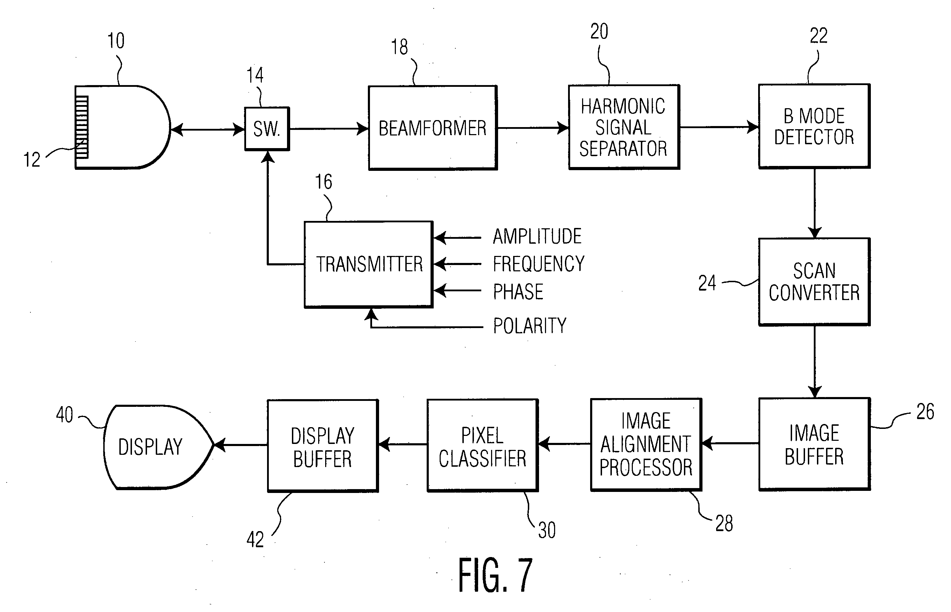

It will be appreciated that the sensitivity and accuracy of many of these techniques will be dependent upon being able to reliably receive a sequence of signal data from the same image point over a period of time so that the temporal data from the same point will be analyzed and suspect locations accurately denoted in the parametric image. This capability will be compromised in the presence of motion, including probe motion and patient motion such as that resulting from breathing. An ultrasound system which addresses this problem is shown in FIGURE 7, which is a variation of FIGURE 1 in which like reference numbers denote the same functionality in the two drawings. The embodiment of FIGURE 7 contains an image alignment processor 28 which operates to spatially align successive images in a sequence of acquired images, thereby reducing or eliminating the image-to-image motional effects. A number of techniques may be employed to realize the image alignment processor, including those shown and described in US Pat. 6,589,176 entitled "Ultrasonic Image Stabilization System and Method," and US Pat. 5,538,004 entitled "Method and Apparatus for Tissue-Centered Scan Conversion In An Ultrasound Imaging System." In general, each of these two approaches extract inter-frame motion by a comparison of successive images or by motional data provided by a device such as a probe motion sensor. In either case, the measured motion is used to align the successive images. It may be preferable to conduct image alignment with fundamental frequency tissue images as there may be times when the harmonic signal levels are too low to confidently align images. In this manner, the same pixel or voxel location in a succession of images will correspond to the same point in the body, thereby producing accurate, spatially correlated temporal data from each location in the field of an image for analysis.

Thus, an embodiment of the present invention can distinguish potential HCC lesions by highlighting early contrast wash-in areas in a two or three dimensional ultrasound image, as compared to normal tissue and benign structures such as hemangiomas which often fill more slowly with contrast. In a typical diagnosis potential HCC lesions will brighten with contrast first, and normal liver tissue will brighten some 10-20 seconds later, with hemangiomas generally taking a full 120 seconds to fill and brighten.

## Claims

1. An ultrasonic diagnostic imaging system comprising:
a probe (10) configured act to transmit and receive signals from a region of the body where time of arrival of a contrast agent is characteristic of a disease state;
a detector (20) configured detect characteristic signals received from a contrast agent;
an image processor (24) responsive to the detector (20);
a pixel classifier (30), responsive to the image processor (24), configured identify signals from the early wash-in of contrast agent, wherein the pixel classifier further comprises a time of arrival detector (52) and a timer (56) and is configured to classify pixel or voxel data received from a sequence of images by a threshold level and a time of reception so that the time of arrival detector (52) is operative during a time interval when the timer (56) is running, the timer (56) being initiated the first time when a pixel or voxel value exceeds the threshold and times out before late arrival of the contrast agent;
a parametric image processor responsive to the pixel classifier (30), which produces an ultrasound image in which an area of early contrast agent arrival is highlighted; and
an image display (40) for displaying the parametric image.

2. The ultrasonic diagnostic imaging system of Claim 1, wherein the probe (10) acts to transmit and receive ultrasonic signals to and from the liver where time of arrival of a contrast agent may be characteristic of a liver lesion.

3. The ultrasonic diagnostic imaging system of Claim 1, wherein the pixel classifier (30) further classifies pixel or voxel data received from a sequence of images by a curve-fitting process.

4. The ultrasonic diagnostic imaging system of Claim 1, wherein the detector (20) comprises a nonlinear signal component detector.

5. The ultrasonic diagnostic imaging system of Claim 4, wherein the nonlinear signal component detector comprises a harmonic signal detector.

6. The ultrasonic diagnostic imaging system of Claim 4, wherein the nonlinear signal component detector comprises a pulse inversion signal processor.

7. The ultrasonic diagnostic imaging system of Claim 1, further comprising:
an image alignment processor (28), responsive to a sequence of received images, which acts to spatially align the content of the images.

8. The ultrasonic diagnostic imaging system of Claim 7, wherein the image alignment processor (28) further acts to align the tissue shown in a sequence of images to the same pixel or voxel locations from image to image.

## Patentansprüche

1. Diagnostisches Ultraschall-Bildgebungssystem, das Folgendes umfasst:
einen Schallkopf (10), der konfiguriert ist, um Signale in eine Körperregion zu senden und aus der Körperregion zu empfangen, wobei der Zeitpunkt der Ankunft eines Kontrastmittels charakteristisch für einen Krankheitszustand ist;
einen Detektor (20), der konfiguriert ist, um von einem Kontrastmittel empfangene charakteristische Signale zu detektieren;
einen auf den Detektor (20) reagierenden Bildprozessor (24);
einen auf den Bildprozessor (24) reagierenden Pixelklassifizierer (30), der konfiguriert ist, um Signale aus dem frühen Einschwemmen des Kontrastmittels zu identifizieren, wobei der Pixelklassifizierer weiterhin einen Ankunftszeit-Detektor (52) und einen Timer (56) umfasst und konfiguriert ist, um aus einer Bildsequenz empfangene Pixel- oder Voxeldaten nach einem Schwellenwertpegel und einer Empfangszeit zu klassifizieren, so dass der Ankunftszeit-Detektor (52) während eines Zeitintervalls in Funktion ist, wenn der Timer (56) läuft, wobei der Timer (56) zum ersten Mal initiiert wird, wenn ein Pixel- oder Voxelwert den Schwellenwert überschreitet, und vor der späten Ankunft des Kontrastmittels abläuft;
einen auf den Pixelklassifizierer (30) reagierenden parametrischen Bildprozessor, der ein Ultraschallbild erzeugt, in dem ein Bereich einer frühen Kontrastmittelankunft hervorgehoben ist; und
eine Bildanzeige (40) zum Anzeigen des parametrischen Bildes.

2. Diagnostisches Ultraschall-Bildgebungssystem nach Anspruch 1, wobei der Schallkopf (10) agiert, um Ultraschallsignale zu der Leber zu senden und von der Leber zu empfangen, wobei die Ankunftszeit eines Kontrastmittels für eine Leberläsion charakteristisch sein kann.

3. Diagnostisches Ultraschall-Bildgebungssystem nach Anspruch 1, wobei der Pixelklassifizierer (30) weiterhin aus einer Bildsequenz empfangene Pixel- oder Voxeldaten nach einem Kurvenanpassungsprozess klassifiziert.

4. Diagnostisches Ultraschall-Bildgebungssystem nach Anspruch 1, wobei der Detektor (20) einen Dektetor für nichtlineare Signalkomponenten umfasst.

5. Diagnostisches Ultraschall-Bildgebungssystem nach Anspruch 4, wobei der Detektor für nichtlineare Signalkomponenten einen Detektor für harmonische Signale umfasst.

6. Diagnostisches Ultraschall-Bildgebungssystem nach Anspruch 4, wobei der Detektor für nichtlineare Signalkomponenten einen Impulsumkehr-Signalprozessor umfasst.

7. Diagnostisches Ultraschall-Bildgebungssystem nach Anspruch 1, das weiterhin Folgendes umfasst:
einen auf eine Sequenz empfangener Bilder reagierenden Bildausrichtungsprozessor (28), der agiert, um den Inhalt der Bilder räumlich auszurichten.

8. Diagnostisches Ultraschall-Bildgebungssystem nach Anspruch 7, wobei der Bildausrichtungsprozessor (28) weiterhin agiert, um das in einer Bildsequenz gezeigte Gewebe von Bild zu Bild auf die gleichen Pixel- oder Voxelorte auszurichten.

## Revendications

1. Système d'imagerie diagnostique ultrasonique, comprenant :
une sonde (10) configurée pour agir pour émettre et recevoir des signaux à partir d'une région du corps où l'instant d'arrivée d'un produit de contraste est caractéristique d'un état pathogène ;
un détecteur (20) configuré pour détecter des signaux caractéristiques reçus à partir d'un produit de contraste ;
un processeur d'image (24) répondant au détecteur (20) ;
un classifieur de pixel (30), répondant au processeur d'image (24), configuré pour identifier des signaux à partir de l'éclaircissement précoce de produit de contraste, dans lequel le classifieur de pixel comprend en outre un détecteur d'instant d'arrivée (52) et une minuterie (56) et est configuré pour classifier des données de pixel ou de voxel reçues à partir d'une séquence d'images selon un niveau de seuil et l'instant de réception pour que le détecteur d'instant d'arrivée (52) soit fonctionnel durant un intervalle de temps lorsque la minuterie (56) est en fonctionnement, la minuterie (56) étant commencée la première fois qu'une valeur de pixel ou de voxel dépasse le seuil et s'arrête avant l'arrivée tardive du produit de contraste ;
un processeur d'image paramétrique répondant au classifieur de pixel (30), qui produit une image ultrasonore dans laquelle une zone d'arrivée précoce de produit de contraste est mise en valeur ; et
un affichage d'image (40) pour afficher l'image paramétrique.

2. Système d'imagerie diagnostique ultrasonique selon la revendication 1, dans lequel la sonde (10) agit pour émettre et recevoir des signaux ultrasoniques vers le foie et à partir de celui-ci, où l'instant d'arrivée d'un produit de contraste peut être caractéristique d'une lésion hépatique.

3. Système d'imagerie diagnostique ultrasonique selon la revendication 1, dans lequel le classifieur de pixel (30) classifie en outre des données de pixel ou de voxel reçues à partir d'une séquence d'images par un procédé d'ajustement de courbe.

4. Système d'imagerie diagnostique ultrasonique selon la revendication 1, dans lequel le détecteur (20) comprend un détecteur de composant de signal non linéaire.

5. Système d'imagerie diagnostique ultrasonique selon la revendication 4, dans lequel le détecteur de composant de signal non linéaire comprend un détecteur de signal harmonique.

6. Système d'imagerie diagnostique ultrasonique selon la revendication 4, dans lequel le détecteur de composant de signal non linéaire comprend un processeur de signal d'inversion d'impulsion.

7. Système d'imagerie diagnostique ultrasonique selon la revendication 1, comprenant en outre :
un processeur d'alignement d'image (28), répondant à une séquence d'images reçues, qui agit pour aligner spatialement le contenu des images.

8. Système d'imagerie diagnostique ultrasonique selon la revendication 7, dans lequel le processeur d'alignement d'image (28) agit en outre pour aligner le tissu représenté dans une séquence d'images sur les mêmes localisations de pixel ou de voxel d'une image à l'autre.
